# EUROPEAN PATENT APPLICATION

(11) **EP 4 578 373 A1**
(43) Date of publication of application: **02.07.2025**
(21) Application number: 23857129.3
(22) Date of filing: 01.08.2023
(51) Int. Cl.: A61B 1/06

(54) **PROCESSOR FOR ELECTRONIC ENDOSCOPES, AND ELECTRONIC ENDOSCOPE SYSTEM**

(30) Priority: 25.08.2022 JP 2022134492
(71) Applicant: Hoya Corporation, Tokyo 160-8347 (JP)
(72) Inventor: SHIMOTASHIRO Shinya, Tokyo 160-8347 (JP)
(74) Representative: Schaumburg und Partner Patentanwälte mbB
(86) International application number: PCT/JP2023/028058
(87) International publication number: WO 2024/043014

(57) **Abstract**

One aspect of the present invention provides a processor for electronic endoscope, the processor being used in an electronic endoscope including an image sensor that is configured to capture an image of a biological tissue, the processor including: a light source unit that synthesizes light beams to be emitted from a plurality of light emitting elements to generate illumination light toward the biological tissue; and a current control unit that controls a current to be applied to each of the light emitting elements in the light source unit. The current control unit includes a current generation unit that generates, as a current to be applied to each of the light emitting elements, an output current which has a duty ratio corresponding to a pulse width modulation control signal (PWM control signal) and has an amplitude corresponding to a lower-voltage control signal selected from a first control signal and a second control signal obtained by subjecting the PWM control signal to filter processing, and a feedback circuit unit that feeds back a current flowing through each of the light-emitting elements to stabilize the output current generated by the current generation unit.

## Description

### Technical Field

The present invention relates to a processor for electronic endoscope used in an electronic endoscope including an image sensor configured to capture an image of a biological tissue, and an electronic endoscope system.

### Background Art

In a medical equipment field, there is known an endoscope system capable of generating an image suitable for diagnosing a lesion hidden in a body cavity, by illuminating a biological tissue in the body cavity and imaging the illuminated biological tissue in the body cavity as an object.

Conventionally, a lamp light source such as a xenon lamp or a halogen lamp that emits white light has been used as illumination light. Recently, a semiconductor light source having a light emitting element such as a light emitting diode (LED) or a laser diode (LD) that emits light having a specific wavelength band has been used instead of the lamp light source.

For example, JP 2017-60860 A discloses an endoscope system including an endoscope having an illumination optical system that irradiates a subject with light from a light source and an imaging optical system including an image sensor that images the subject, and a control device to which the endoscope is detachably connected. This system has a plurality of types of control patterns representing a relationship between a light amount instruction value and a control output value to a light source, and a light source control unit switches to one of the control patterns on the basis of an identification result of a type of the image sensor and controls the intensity of light emitted from the light source on the basis of the switched control pattern.

### Summary of Invention

### Technical Problem

Meanwhile, a driver integrated circuit (IC) that drives an LED may include both a pulse width modulation (PWM) dimming circuit and an analog dimming circuit, and may further include a feedback circuit in order to stabilize an output current to be applied to the LED. Such a driver IC is configured not to selectively execute control based on either a PWM dimming method or an analog dimming method, but to simultaneously execute the control based on the PWM dimming method and the control based on the analog dimming method.

When such a driver IC for LED is used in the electronic endoscope system, the LED may be destroyed due to a high level of the frequency of a PWM signal handled in the electronic endoscope system. For example, in order to capture an image at a high shutter speed (for example, 1/1000 second) at which a still image of an object does not have a blur under irradiation light with PWM dimming, it is required to drive the LED via PWM at a speed 10 to 100 times higher than the high shutter speed (for example, to apply a PWM current of 10 to 100 kHz to the LED). However, such a high frequency PWM current cannot be stably controlled due to the response delay of a feedback system, and thus, the peak of the output current may exceed the rated value of the LED, resulting in that the LED may be destroyed.

In view of this, an object of the present invention is to protect an LED when a processor for electronic endoscope including a light source having a light emitting element performs diming control on the light emitting element with pulse width modulation.

### Solution to Problem

One aspect of the present disclosure provides a processor for electronic endoscope, the processor being used in an electronic endoscope including an image sensor that is configured to capture an image of a biological tissue, the processor including:
a light source unit that synthesizes light beams to be emitted from a plurality of light emitting elements to generate illumination light toward the biological tissue; and
a current control unit that controls a current to be applied to each of the light emitting elements in the light source unit.

The current control unit includes:
a current generation unit that generates, as a current to be applied to each of the light emitting elements, an output current which has a duty ratio corresponding to a pulse width modulation control signal (PWM control signal) and has an amplitude corresponding to a lower-voltage control signal selected from a first control signal and a second control signal obtained by subjecting the PWM control signal to filter processing; and
a feedback circuit unit that feeds back a current flowing through each of the light-emitting elements to stabilize the output current generated by the current generation unit.

The signal level of the second control signal may be adjusted so that the signal level of the second control signal at a duty ratio of 100% of the PWM control signal is higher than the maximum value of a signal level of the first control signal.

The processor for electronic endoscope may limit a use range of a duty ratio of the PWM control signal according to the signal level of the first control signal such that the signal level of the first control signal is lower than the signal level of the second control signal.

The current control unit may include a low-pass filter circuit having a cutoff frequency corresponding to a frequency of the PWM control signal as a circuit that performs the filtering processing.

Another aspect of the present disclosure provides an electronic endoscope system including:
the processor for electronic endoscope described above; and
an electronic endoscope that is connected to the processor for electronic endoscope and includes an image sensor configured to acquire a captured image of the biological tissue.

### Advantageous Effects of Invention

According to the processor for electronic endoscope and the electronic endoscope system described above, an LED in the processor for electronic endoscope including a light source having a light emitting element can be protected when dimming control is performed on the light emitting element with pulse width modulation.

### Brief Description of Drawings

Fig. 1 is a block diagram illustrating an example of the configuration of an electronic endoscope system according to an embodiment.
Fig. 2 is a block diagram illustrating the configuration of a light source device according to a reference example.
Fig. 3 is a timing chart illustrating operation of the light source device illustrated in Fig. 2.
Fig. 4 is a block diagram illustrating the configuration of a light source device according to the embodiment.
Fig. 5 is a diagram illustrating a configuration example of a filter circuit included in the light source device in Fig. 4.
Fig. 6 is a timing chart illustrating operation of the light source device illustrated in Fig. 4.
Fig. 7 is a timing chart illustrating an actual waveform of the light source device illustrated in Fig. 4.
Fig. 8 is a diagram illustrating an example of a relationship between a voltage level of a control signal and a settable duty ratio of a PWM signal in analog dimming.

### Description of Embodiments

An electronic endoscope system according to the present embodiment will be described below in detail with reference to the drawings.

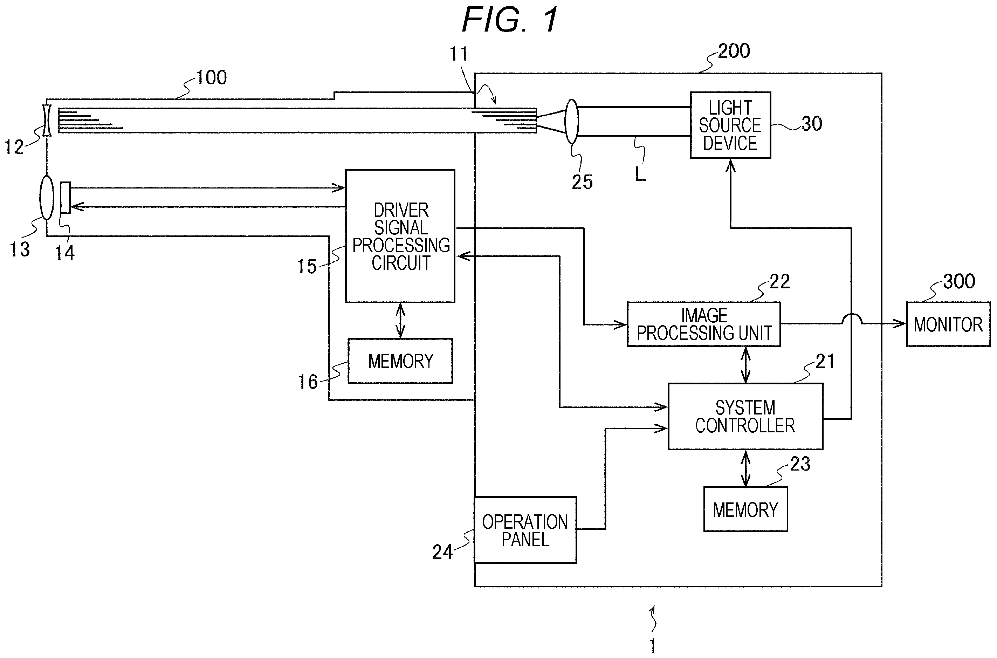

Fig. 1 is a block diagram illustrating an example of the configuration of an electronic endoscope system 1 according to the present embodiment. As illustrated in Fig. 1, the electronic endoscope system 1 is specialized for medical use and includes an electronic scope (endoscope) 100, a processor 200, and a monitor 300.

The processor 200 includes a system controller 21. The system controller 21 executes various programs stored in a memory 23 and controls the entire electronic endoscope system 1 in an integrated manner. Furthermore, the system controller 21 is connected to an operation panel 24. The system controller 21 changes operations of the electronic endoscope system 1 and a parameter for each of the operations in accordance with an operator's instruction input to the operation panel 24. The system controller 21 outputs a clock pulse for adjusting an operation timing of each unit to the corresponding circuit in the electronic endoscope system 1.

The processor 200 includes a light source device 30. The light source device 30 emits illumination light L for illuminating an object such as a biological tissue in a body cavity. The illumination light L includes white light, pseudo white light, or special light. According to the embodiment, it is preferable that the light source device 30 selects one of a mode of constantly emitting the white light or the pseudo white light as the illumination light L and a mode of alternately emitting the white light or pseudo white light and the special light as the illumination light L, and emits the white light, the pseudo white light, or the special light based on the selected mode. The white light is light having a flat spectral intensity distribution in a visible light band, and the pseudo white light is light which is a mixture of light of a plurality of wavelength bands and has non-flat spectral intensity distribution. The special light is light of a narrow wavelength band, such as blue or green, in the visible light band. The light of the blue or green wavelength band is used at the time of enhancing a specific portion of the biological tissue for observation. The illumination light L emitted from the light source device 30 is condensed onto an incident end face of a light carrying bundle (LCB) 11 by a condenser lens 25, and enters the LCB 11.

The illumination light L entering the LCB 11 propagates through the LCB 11. The illumination light L propagating through the LCB 11 is emitted from an exit end face of the LCB 11 disposed at a distal end of the electronic scope 100, and irradiates the object via a light distribution lens 12. Return light from the object illuminated with the illumination light L from the light distribution lens 12 forms an optical image on a light receiving surface of a solid-state image sensor 14 via an objective lens 13.

The solid-state image sensor 14 is a single-chip color charge coupled device (CCD) image sensor having a Bayer pixel arrangement. The solid-state image sensor 14 accumulates an optical image formed by each of pixels on the light receiving surface as charges corresponding to the amount of light, and generates and outputs image signals of red (R), green (G), and blue (B). Note that the solid-state image sensor 14 is not limited to a CCD image sensor, and may be replaced with a complementary metal oxide semiconductor (CMOS) image sensor or other types of imaging devices. Furthermore, the solid-state image sensor 14 may include a complementary color filter.

A driver signal processing circuit 15 is provided in a connection portion of the electronic scope 100. An image signal of the object is input to the driver signal processing circuit 15 from the solid-state image sensor 14 in a predetermined frame cycle. The frame cycle is, for example, 1/30 seconds. The driver signal processing circuit 15 performs predetermined processing including A/D conversion on the image signal input from the solid-state image sensor 14 and outputs the processed image signal to an image processing unit 22 of the processor 200.

The image processing unit 22 performs predetermined image processing to be described later to generate a video format signal, and outputs the video format signal to the monitor 300.

Furthermore, the driver signal processing circuit 15 accesses a memory 16 and reads specific information regarding the electronic scope 100. The specific information regarding the electronic scope 100 recorded in the memory 16 includes, for example, the number of pixels and sensitivity of the solid-state image sensor 14, a frame rate with which the electronic scope 100 is operable, and a model number. The driver signal processing circuit 15 outputs the specific information read from the memory 16 to the system controller 21. The specific information may include information specific to the solid-state image sensor 14 such as the number of pixels and resolution of the solid-state image sensor 14, and information regarding an optical system such as an angle of view, a focal length, and a depth of field.

The system controller 21 performs various types of calculation based on the specific information regarding the electronic scope 100 to generate a control signal. The system controller 21 controls the operation and timing of each circuit in the processor 200 using the generated control signal so that processing suitable for the electronic scope 100 connected to the processor 200 is performed.

The system controller 21 supplies a clock pulse to the driver signal processing circuit 15. The driver signal processing circuit 15 controls driving of the solid-state image sensor 14 at a timing synchronized with the frame rate of a video image processed by the processor 200 in accordance with the clock pulse supplied from the system controller 21.

Next, the light source device 30 built in the processor 200 will be described.

Commonly, a light source device uses a plurality of LEDs as a light source to obtain white illumination light, and light beams emitted from the plurality of LEDs are synthesized. For example, three LEDs that are a blue LED, a green LED, and a red LED may be used as the light source, and five LEDs that are an ultra violet (UV) LED, a blue LED, a green LED, an amber LED, and a red LED may be used as the light source.

Each LED can perform analog dimming and PWM dimming. The analog dimming refers to controlling the intensity of light emitted from the LED according to the magnitude of a current applied to the LED. On the other hand, the PWM dimming refers to using a current with a pulse width modulation (PWM) waveform as a current applied to the LED and controlling the intensity of light emitted from the LED with the duty ratio of PWM.

The light source device 30 is configured to be capable of independently executing analog dimming and PWM dimming.

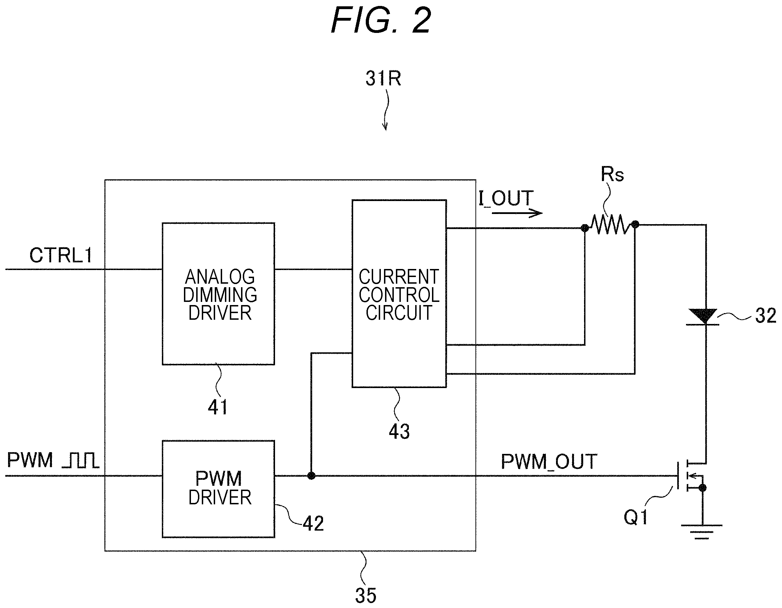

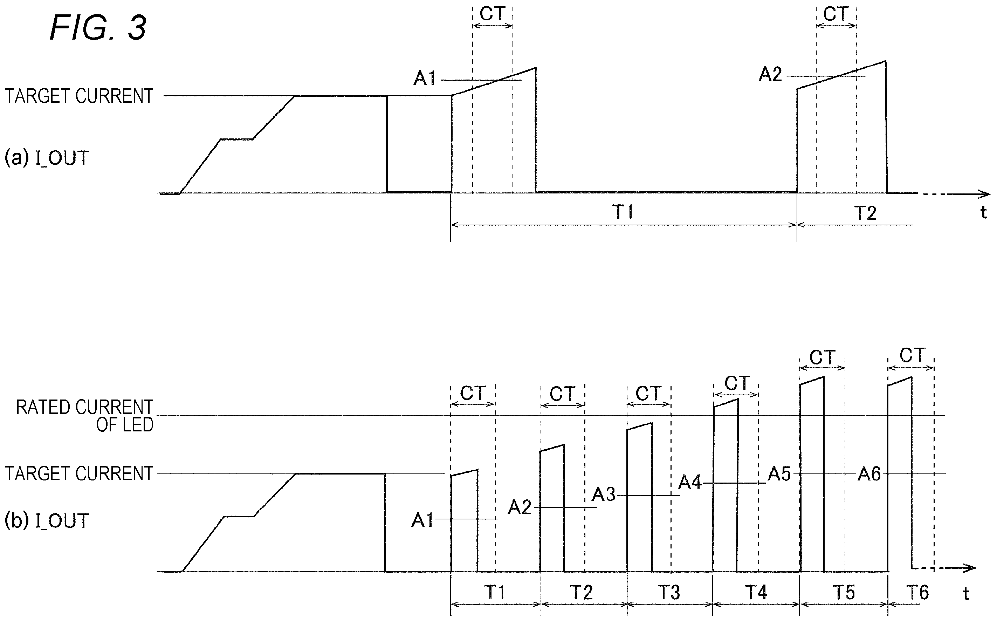

Prior to describing the configuration of the light source device 30 according to the present embodiment, a reference example of the light source device will be described below with reference to Figs. 2 and 3 for facilitating understanding of the light source device 30 according to the present embodiment.

Fig. 2 is a block diagram illustrating the configuration of a light source device according to the reference example. Fig. 3 is a timing chart illustrating operation of the light source device according to the reference example. Fig. 2 illustrates only the configuration of the light source device for a single LED 32, although the light source device includes a plurality of LEDs as described above.

Referring to Fig. 2, the light source device according to the reference example includes an LED control unit 31R that controls a current to be applied to the LED 32. The LED control unit 31R includes an LED driver circuit 35, a sense resistor Rs, and a MOS transistor Q1.

The LED driver circuit 35 receives a control signal CTRL1 and a PWM signal (PWM) supplied from the system controller, and outputs a PWM output PWM_OUT for controlling the MOS transistor Q1 and an output current I_OUT to be applied to the LED 32. The LED driver circuit 35 includes, for example, a hardware module including a field-programmable gate array (FPGA).

The LED driver circuit 35 includes an analog dimming driver 41, a PWM driver 42, and a current control circuit 43.

The analog dimming driver 41 generates a current that varies according to the voltage level of the control signal CTRL1 and outputs the current to the current control circuit 43.

The PWM driver 42 generates a PWM output PWM_OUT obtained by amplifying the PWM signal to a voltage capable of driving the MOS transistor Q1, supplies the generated PWM output PWM_OUT to the gate of the MOS transistor Q1, and outputs the PWM output PWM_OUT to the current control circuit 43.

The current control circuit 43 amplifies the current output from the analog dimming driver 41 and generates the output current I_OUT based on the duty ratio of the PWM output PWM_OUT supplied from the PWM driver 42. The output current I_OUT generated by the current control circuit 43 is based on both the voltage level of the control signal CTRL1 and the duty ratio of the PWM signal. Therefore, the output current I_OUT has a pulse waveform determined by the duty ratio of the PWM signal, and the amplitude of the pulse waveform has a value corresponding to the voltage level of the control signal CTRL1.

When analog dimming is performed, the duty ratio of the PWM signal is set to 100%, and the voltage level of the control signal CTRL1 is made variable. That is, when the duty ratio of the PWM signal is 100%, the PWM output PWM_OUT is in a H level, so that the MOS transistor Q1 is turned on, and the output current I_OUT corresponding to the amplitude of the control signal CTRL1 flows through the LED 32.

When PWM dimming is performed, the voltage level of the control signal CTRL1 is made constant, and the duty ratio of the PWM signal is made variable. In that case, the output current I_OUT has a constant value, and the MOS transistor Q1 is turned on/off according to the level of the PWM output PWM_OUT having the same duty ratio as the duty ratio of the PWM signal, so that the output current I_OUT based on the duty ratio of the PWM signal flows through the LED 32.

The current control circuit 43 also detects a current (output current I_OUT) flowing through the LED 32 using a voltage drop in the sense resistor Rs, and performs feedback control for stabilizing the output current I_OUT on the basis of the detection result. This feedback control is performed such that the average output current I_OUT for a predetermined time converges to a target current as a controlled variable.

Fig. 3(a) is a timing chart illustrating an output current I_OUT (LED current) when a PWM signal having a frequency sufficiently lower than the response characteristics of the feedback control in the current control circuit 43 is input to the LED driver circuit 35. In Fig. 3, T1, T2,... each correspond to one cycle, and CT is a predetermined time for determining the controlled variable.

As illustrated in Fig. 3(a), when a PWM signal having a relatively low frequency is input to the LED driver circuit 35, the average output current I_OUT can be made substantially equal to the target current due to the pulse width being longer than the predetermined time CT even in a case where the duty ratio is small. That is, the amplitude levels A1, A2,... of the average output current I_OUT in the predetermined time CT in the cycles T1, T2,... are substantially close to the target current, and the output current I_OUT can converge to the target current in a timely manner. Although the overshoot occurs instantaneously immediately after the rise of the pulse of the output current I_OUT, the output current I_OUT can converge to the target current within the pulse width because the pulse width is relatively long.

Fig. 3(b) is a timing chart illustrating an output current I_OUT (LED current) when a PWM signal having a frequency sufficiently higher than the response characteristics of the feedback control in the current control circuit 43 is input to the LED driver circuit 35.

As illustrated in Fig. 3(b), when a PWM signal having a high frequency and a small duty ratio is input to the LED driver circuit 35, the average output current I_OUT set as the controlled variable does not reflect the amplitude of the pulse and has a value lower than the amplitude of the pulse, due to the pulse width being shorter than the predetermined time CT when the target current is set. In Fig. 3(b), average output currents I_OUT set as controlled variables in the cycles T1 to T6 are A1 to A6, respectively.

The average output current I_OUT (controlled variable) within the predetermined time CT is low regardless of the fact that the amplitude of the pulse of the output current I_OUT substantially coincides with that of the target current in the cycle T1 in Fig. 3(b), and thus, the LED driver circuit 35 generates the output current I_OUT so that the amplitude of the pulse increases in order to bring the controlled variable close to the target current in the cycle T2. Similarly, the output current I_OUT in which the amplitude of the pulse gradually increases is generated in each of the cycles T3 to T5. In the cycle T5, the average output current I_OUT within the predetermined time CT substantially coincides with the target current, but at this time point, the amplitude of the pulse of the output current I_OUT exceeds the rated current of the LED, resulting in that the LED 32 may be broken.

In addition, when a PWM signal having a high frequency and a small duty ratio is input to the LED driver circuit 35, there is also a problem that the overshoot immediately after the rise of the pulse of the output current I_OUT cannot converge within a short pulse width.

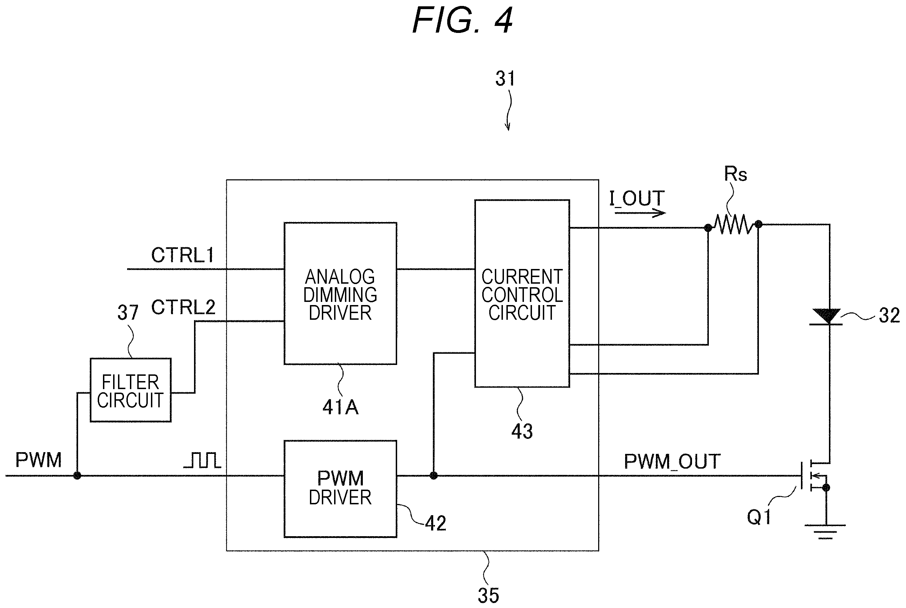

Next, the configuration of the light source device 30 according to the present embodiment will be described with reference to Fig. 4.

Fig. 4 is a block diagram illustrating the configuration of the light source device 30 according to the embodiment. As can be seen by comparing Figs. 2 and 4, the LED control unit 31 of the light source device 30 is different from the light source device according to the reference example in that a filter circuit 37 is added.

The filter circuit 37 receives the PWM signal and outputs a control signal CTRL2 to an analog dimming driver 41A. The analog dimming driver 41A generates a current based on a lower-voltage signal selected from the control signals CTRL1 and CTRL2 and outputs the current to the current control circuit 43. That is, the LED control unit 31 performs analog dimming on the basis of the lower-voltage control signal selected from the control signals CTRL1 and CTRL2.

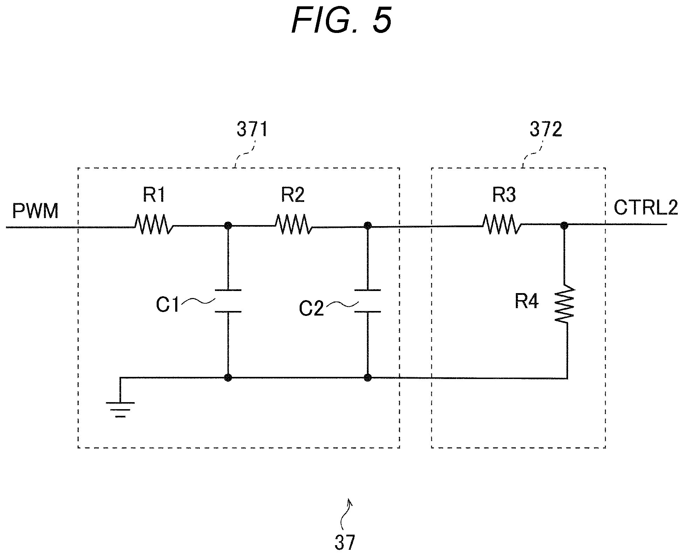

Fig. 5 illustrates an example of the filter circuit 37.

In the example illustrated in Fig. 5, the filter circuit 37 includes an integration circuit 371 and a voltage divider 372. The integration circuit 371 includes resistors R1 and R2 and capacitors C1 and C2, and constitutes a low-pass filter circuit having a cutoff frequency (for example, 2 to 3 kHz) corresponding to the frequency of the PWM control signal. The voltage divider 372 includes resistors R3 and R4 for dividing the output voltage of the integration circuit 371, and constitutes a voltage dividing circuit for adjusting the DC level of the control signal CTRL2.

The specific circuit illustrated in Fig. 5 is merely an example, and a person skilled in the art could set, as appropriate, a low-pass filter having a cutoff frequency that is necessary.

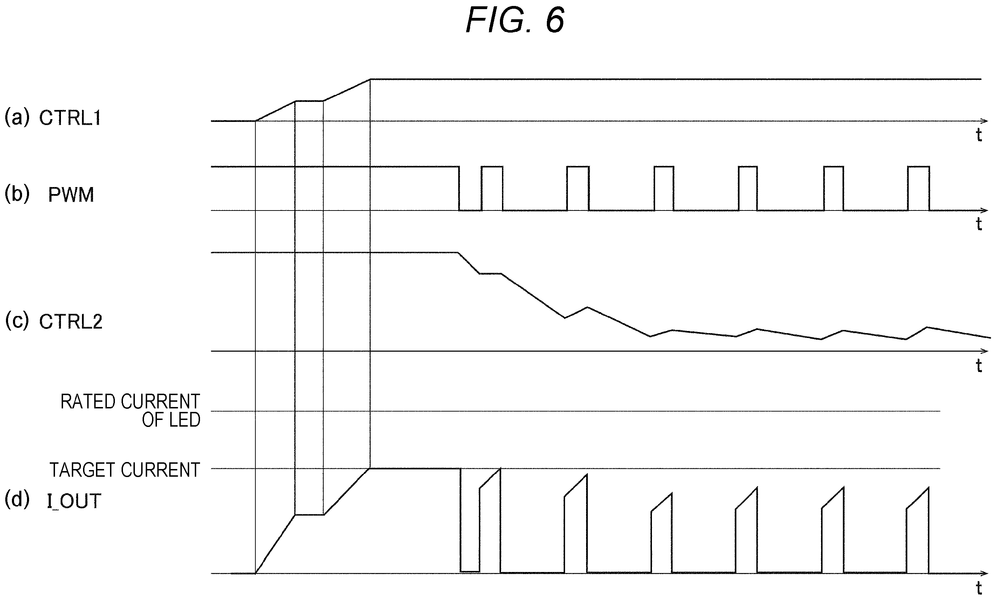

In the LED control unit 31, the filter circuit 37 and the analog dimming driver 41A provide an LED protective function that prevents the amplitude of the pulse of the output current I_OUT from exceeding the rated current of the LED 32 when a PWM signal having a high frequency and a small duty ratio is input to the LED driver circuit 35. The LED protective function will be described below with reference to the timing chart of Fig. 6.

In the timing chart of Fig. 6, (a) indicates the control signal CTRL1, (b) indicates the PWM signal, (c) indicates the control signal CTRL2, and (d) indicates the output current I_OUT.

In Fig. 6, when a PWM signal having a frequency higher than the cutoff frequency set in the filter circuit 37 and a small duty ratio is input to the LED driver circuit 35, the filter circuit 37 attenuates a high frequency component of the PWM signal and outputs a control signal CTRL2 having a frequency component close to DC to the analog dimming driver 41A as illustrated in Fig. 6(c).

The voltage level of the control signal CTRL2 having a frequency component close to DC is lower than that of the control signal CTRL1. Therefore, the analog dimming driver 41A generates a current based on a lower-voltage signal selected from the control signals CTRL1 and CTRL2 and outputs the current to the current control circuit 43, whereby analog dimming is performed based on the control signal CTRL2. As a result, the amplitude of the pulse of the output current I_OUT generated by the current control circuit 43 is suppressed to be low and does not exceed the rated current of the LED 32 as illustrated in Fig. 6(d), whereby the LED 32 is protected. Although the amplitude of the pulse of the output current I_OUT at this time does not reach the target current and dimming cannot be performed, the LED 32 can be protected from unintended overcurrent.

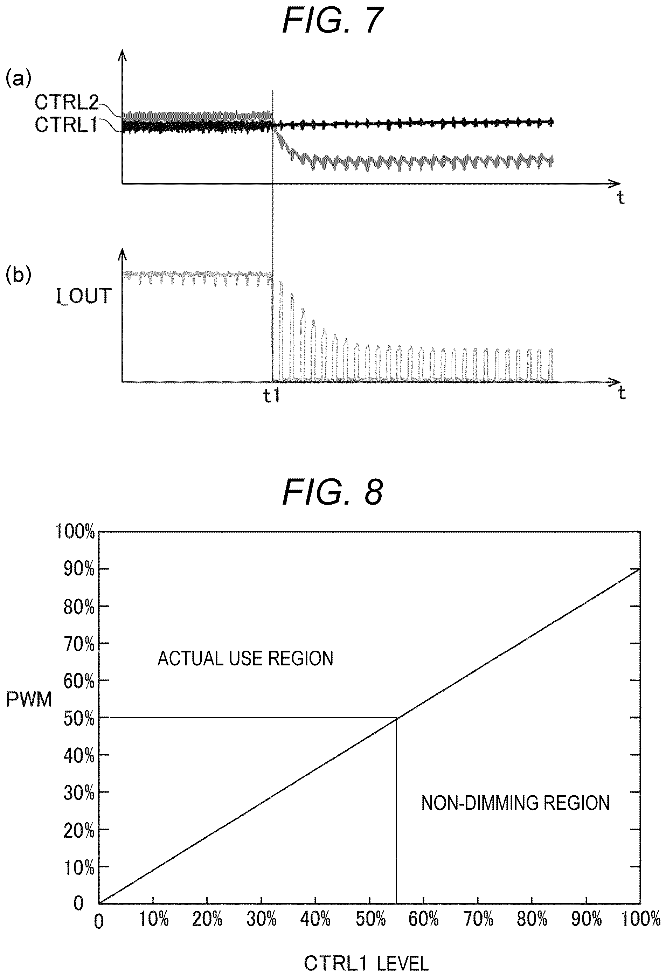

Fig. 7 is a timing chart illustrating an actual waveform of the light source device 30 illustrated in Fig. 4.

In Fig. 7, (a) indicates waveforms of the control signals CTRL1 and CTRL2, and (b) indicates a waveform of the output current I_OUT.

In Fig. 7, it is assumed that analog dimming (that is, the duty ratio of the PWM signal is 100%) is performed based on only the control signal CTRL1 before time t1, and a PWM signal having a high frequency and a small duty ratio is input to the LED driver circuit 35 after time t1. In that case, after time t1, the control signal CTRL2 is converted into a signal having a lower voltage and a lower frequency component than the control signal CTRL1 by attenuating the high-frequency component of the PWM signal. Then, the analog dimming driver 41A performs analog dimming on the basis of the control signal CTRL2 after time t1. As a result, the pulse amplitude of the output current I_OUT gradually decreases like the control signal CTRL2 as illustrated in Fig. 7(b), and thus, the LED 32 is protected.

As described above, the electronic endoscope system 1 according to the present embodiment includes an LED control unit 31 (an example of a current control unit) configured to be able to simultaneously execute both analog dimming and PWM dimming for each LED of the light source device 30. The LED control unit 31 receives a control signal CTRL1 (an example of a first control signal) for performing analog dimming, a PWM signal, and a control signal CTRL2 (an example of a second control signal) obtained by performing filter processing on the PWM signal. The LED control unit 31 generates an output current having an amplitude corresponding to a lower-voltage signal selected from the control signals CTRL11 and CTRL2. The duty ratio of the output current is determined by the duty ratio of the PWM signal. The LED control unit 31 (an example of a feedback circuit unit) also performs feedback control for stabilizing the output current by feeding back the current flowing through each LED.

In the above configuration, when a PWM signal having a high frequency and a small duty ratio is input, the control signal CTRL2 is converted into a signal having a frequency component close to DC by attenuating a high frequency component of the PWM signal, and the voltage level of the control signal CTRL2 is lower than that of the control signal CTRL1. Therefore, analog dimming is performed based on the control signal CTRL2 having a lower voltage level. Accordingly, even when the feedback control is performed so as to converge the average output current I_OUT for the predetermined time to the target current as the controlled variable, the output current I_OUT does not exceed the rated current of the LED, and thus the LED is protected.

In the light source device 30 according to the present embodiment, the above-described protective function is to protect the LED 32 when a PWM signal having an extremely high frequency and a small duty ratio is input. However, in a case where such an unusual PWM signal is not input, it is preferable to perform analog dimming based on the control signal CTRL1. From this viewpoint, before time t1 (that is, before the protective function works), the control signal CTRL2 is preferably higher than the control signal CTRL1 as illustrated in Fig. 7. That is, when analog dimming is performed, it is preferable to set the voltage of the control signal CTRL2 higher than the voltage of the control signal CTRL1 so as not to be affected by the control signal CTRL2. Although not limited, the voltage of the control signal CTRL2 is set to be higher than the voltage of the control signal CTRL1 by about 10 to 20%, for example. The value of the voltage of the control signal CTRL2 is adjusted by the voltage divider 372 in the example of the filter circuit 37 in Fig. 5.

In short, in the embodiment, the signal level of the control signal CTRL2 is adjusted such that the signal level of the control signal CTRL2 when the duty ratio of the PWM signal is 100% is higher than the maximum value of the signal level of the control signal CTRL1.

As described above, when the protective function works, the output current I_OUT cannot be controlled to the target current, and the dimming based on the control signal CTRL1 cannot be performed. Therefore, when it is desired to continue the intended dimming operation, it is necessary not to perform PWM dimming in a state where the voltage level of the control signal CTRL1 is high. That is, when the intensity of light emitted from the LED 32 is decreased, it is preferable to first decrease the voltage level of the control signal CTRL1 to perform dimming, and adjust the duty ratio of the PWM signal only when the voltage of the control signal CTRL1 sufficiently decreases.

Fig. 8 illustrates an example of the relationship between the voltage level (CTRL1 level) of the control signal CTRL1 and the settable duty ratio of the PWM signal in the analog dimming.

Note that a case where the voltage of the control signal CTRL2 having the duty ratio of 100% is 10% higher than the control signal CTRL1 when the CTRL1 level is the maximum value (100%) is assumed as an example. In this case, when the duty ratio of the PWM signal is within a range of about 90% to 100%, CTRL2 > CTRL1 is satisfied, so that analog dimming based on the control signal CTRL1 is possible. When the voltage level of the control signal CTRL1 is low, the duty ratio of the PWM signal for enabling analog dimming based on the control signal CTRL1 with the relationship of CTRL2 > CTRL1 being maintained is eased. For example, when the voltage level of the control signal CTRL1 is about 55%, the duty ratio can be set variable within a range of 50% to 100% (the region in this range is defined as "actual use region") in order to maintain the relationship of CTRL2 > CTRL1.

In Fig. 8, the "actual use region" is defined as a region where analog dimming based on the control signal CTRL1 is enabled. When CTRL2 < CTRL1 is satisfied, analog dimming based on the control signal CTRL1 cannot be performed, and thus the region where CTRL2 < CTRL1 is satisfied is defined as "non-dimming region".

Therefore, in the embodiment, the use range of the duty ratio of the PWM signal is limited according to the signal level of the control signal CTRL1 so that the signal level of the control signal CTRL1 is lower than the signal level of the control signal CTRL2. This prevents the analog dimming based on the control signal CTRL1 from becoming impossible due to the duty ratio of the PWM signal.

While the processor for an electronic endoscope and the electronic endoscope system according to the present invention have been described above in detail, the processor for an electronic endoscope and the electronic endoscope system according to the present invention are not limited to the above-described embodiment, and can be modified or altered in various ways without departing from the spirit of the present invention.

The present invention relates to the patent application of Japanese Patent Application No. 2022-134492 filed with the Japan Patent Office on August 25, 2022, the entire contents of which are incorporated herein by reference.

## Claims

1. A processor for electronic endoscope, the processor being used in an electronic endoscope including an image sensor that is configured to capture an image of a biological tissue, the processor comprising:
a light source unit that synthesizes light beams to be emitted from a plurality of light emitting elements to generate illumination light toward the biological tissue; and
a current control unit that controls a current to be applied to each of the light-emitting elements in the light source unit, wherein
the current control unit includes
a current generation unit that generates, as a current to be applied to each of the light emitting elements, an output current which has a duty ratio corresponding to a pulse width modulation control signal (PWM control signal) and has an amplitude corresponding to a lower-voltage control signal selected from a first control signal and a second control signal obtained by subjecting the PWM control signal to filter processing, and
a feedback circuit unit that feeds back a current flowing through each of the light-emitting elements to stabilize the output current generated by the current generation unit.

2. The processor for electronic endoscope according to claim 1, wherein
a signal level of the second control signal is adjusted so that the signal level of the second control signal at a duty ratio of 100% of the PWM control signal is higher than a maximum value of a signal level of the first control signal.

3. The processor for electronic endoscope according to claim 2,
the processor limiting a use range of a duty ratio of the PWM control signal according to the signal level of the first control signal such that the signal level of the first control signal is lower than the signal level of the second control signal.

4. The processor for electronic endoscope according to claim 1, wherein
the current control unit includes a low-pass filter circuit having a cutoff frequency corresponding to a frequency of the PWM control signal as a circuit that performs the filtering processing.

5. An electronic endoscope system comprising:
the processor for electronic endoscope according to any one of claims 1 to 4; and
an electronic endoscope that is connected to the processor for electronic endoscope and includes an image sensor configured to acquire a captured image of the biological tissue.
